# EUROPÄISCHE PATENTSCHRIFT

(11) **EP 0 480 938 B1**
(45) Veröffentlichungstag und Bekanntmachung des Hinweises auf die Patenterteilung: **19.05.1993**
(21) Anmeldenummer: 90908485.7
(22) Anmeldetag: 13.06.1990
(51) Int. Cl.: B28B 1/26, E03D 11/02

(54) **GIESSFORM FÜR EIN TIEFSPÜLKLOSETT MIT GERUCHSVERSCHLUSSZUNGE**
MOULD FOR PRODUCING A FULL-FLUSH WC WITH AN ODOUR-TRAP TONGUE
MOULE DE COULEE POUR UN WATER-CLOSET A NAPPE D'EAU PROFONDE AVEC LANGUE DE SIPHON INODORE

(30) Priorität: 28.06.1989 DE 3921195
(43) Veröffentlichungstag der Anmeldung: 22.04.1992
(73) Patentinhaber: IDEAL-STANDARD GMBH, D-53008 Bonn (DE)
(72) Erfinder: KRAUSE, Gerhard, D-4040 Neuss (DE)
(74) Vertreter: Müller, Karl-Ernst, Dr., Dipl.-Ing.
(86) Internationale Anmeldenummer: DE9000454
(87) Internationale Veröffentlichungsnummer: WO9100170

(56) Entgegenhaltungen:
- FR-A- 2 093 348
- GB-A- 295 821
- US-A- 2 288 991
- US-A- 2 449 249

## Beschreibung

Die Erfindung betrifft eine Gießform zur Herstellung eines Tiefspülklosetts mit einer von der von der Rückwand zum Becken einspringenden Öffnung ausgehenden Geruchsverschlußzunge im Hohlguß mit einem Rückwandkern zur Ausbildung der Öffnung und mit wenigstens einem Formteil zur Ausbildung der Geruchsverschlußzunge.

Bei der Herstellung eines Tiefspülklosetts im Hohlguß bereitet die Ausbildung der Geruchsverschlußzunge besondere Probleme; hierzu ist bekannt, die Gießform mit von ihren Seitenwandungen in das Forminnere vorspringenden und dort die Zungenkontur ausbildenden Formteilen zu versehen, an denen sich nach dem Auffüllen der Gießform mit Schlicker der die Geruchsverschlußzunge ausbildende Scherben bildet, so daß nach dem Ablassen des Schlickers sich die Zunge an den Scherben des Rückwandkerns anschließend gebildet hat. Mit einer bekannten Gießform ist der Nachteil verbunden, daß aufgrund der durch den Austritt der Formteile der Gießform aus dem Scherben gebildeten Löcher in den Seitenwandungen in einem weiteren Verfahrensschritt zur Fertigstellung des Klosetts diese Öffnungen im Wege einer Garnierung geschlossen werden müssen. Durch diesen zusätzlichen Verfahrensschritt ergeben sich neben dem höheren Bearbeitungsaufwand zusätzliche Risiken beim Brennen und hinsichtlich der Qualität der Endprodukte, weil die Garnierung hier eine entsprechende Schwachstelle des Herstellungsprozesses bildet.

Gemäß einer anderen Verfahrensweise ist es bekannt, die Zunge im Vorguß herzustellen und sie in die Gießform zur Durchführung des Hohlgusses beim Klosettkörper einzusetzen. Auch hiermit ist der Nachteil verbunden, daß an der bereits vorgefertigten Zunge beim Hohlguß sich nochmals eine Scherbenbildung vollzieht, womit das nachteilige Herstellungsrisiko verbunden ist, daß sich die Scherbenbildung an dem vorgefertigten, bereits einen Scherben darstellenden Zungenkörper überhaupt nicht beziehungsweise nicht mit der notwendigen Verbindungsqualität vollzieht, so daß der Anteil von Ausschuß bei einem derartigen Verfahren vergleichsweise groß ist.

Aus der US-A-2 449 249 ist weiterhin eine Gießform zur Herstellung eines Klosetts mit einem zungenartigen Teil zur Trennung der Wasserwege bekannt, welches nicht als Tiefspülklosett mit der dadurch bedingten besonderen Form einer Geruchsverschlußzunge ausgebildet ist; bei dieser bekannten Gießform ist ein unmittelbar von der Rückwand beziehungsweise vom Boden des Klosetts her in die Gießform eingesetztes einteiliges Formteil zur Ausbildung des zungenartigen Teils vorgesehen, wobei eine derartige Gießform für die Herstellung eines Tiefspülklosetts mit Geruchsverschlußzunge in der üblichen Ausbildung nicht brauchbar ist.

Der Erfindung liegt daher die Aufgabe zugrunde, eine gattungsgemäße Gießform derart zu verbessern, daß die Herstellung des Tiefspülklosetts in einem einzigen Gießvorgang ohne Nachgarnieren möglich ist.

Die Lösung dieser Aufgabe ergibt sich einschließlich vorteilhafter Ausgestaltungen und Weiterbildungen aus dem Inhalt der Patentansprüche, welche dieser Beschreibung nachgestellt sind.

Die Erfindung sieht in ihrem Grundgedanken vor, daß das Formteil aus mindestens zwei zusammengesetzt der Kontur der Geruchsverschlußzunge entsprechenden Kernteilen unterschiedlicher Abmessung besteht, die in einander angepaßter Formgebung derart ausgebildet sind, daß ein erstes kleineres Kernteil aus dem durch Scherbenbildung gebildeten Zungenhohlraum bei dem dort noch verbleibenden zweiten größeren Kernteil herausziehbar ist, wobei die Berührungsfläche zwischen den Kernteilen eine Richtung des Ausziehweges für die Kernteile verlaufende Krümmung aufweist.

Hiermit ist der Vorteil verbunden, daß sich die Scherbenbildung hinsichtlich der Kontur der Geruchsverschlußzunge an Teilen der Gießform vollzieht, wobei die Formteile nach Bildung des Scherbens aus dem so gebildeten Klosettkörper gezogen werden, so daß eine Nachbearbeitung nicht erforderlich ist, da ein Zugriff auf die Kernteile über die vom Rückwandkern gelassene Öffnung ohne weiteres möglich ist. Damit kann auf jede Nachbehandlung in Form einer Garnierung und auf einen eventuellen Vorguß der Zunge verzichtet werden. Da die Geruchsverschlußzunge in einem Winkel zum den Zugang zu den Kernteilen gebenden Rückwandkern angeordnet ist, können die Kernteile aus dem durch die Scherbenbildung an den Kernteilen entstandenen Hohlraum unter Überwindung des Winkelbereichs zu der durch den Rückwandkern gebildeten Öffnung herausgezogen werden, wobei aufgrund der in Richtung des Ausziehweges der Kernteile gekrümmt ausgebildeten Berührungsfläche das Herausziehen der Kernteile erleichtert ist.

Nach einem Ausführungsbeispiel ist die Länge des zuerst auszuziehenden kleineren Kernteils kürzer bemessen als die Länge des zweiten größeren Kernteils, so daß ein Herausdrehen des kleineren Kernteils aus dem Zungenhohlraum unter Überwindung des zur durch das Ziehen des Rückwandkerns gebildeten Öffnung bestehenden Winkels möglich ist.

Das Herausziehen des ersten kleineren Kernteils wird dadurch erleichtert, daß die dem Rückwandkern zugewandte Fläche der zusammengesetzten Kernteile in einem flacheren Winkel verläuft als die dem Klosettbecken zugewandte Fläche der Zunge, wobei die Fläche des kleineren Kernteils nochmal einen abgesetzten kleineren Winkel zum Rückwandkern aufweisen kann als die Fortsetzungsfläche des größeren Kernteils.

Es ist nach der Erfindung auch möglich, drei oder mehr einzelne Kernteile zu einer Gesamtform für die Geruchsverschlußzunge zusammenzusetzen, solange die Kernteile über die nach Ziehen des Rückwandkerns gegebene Öffnung aus dem Hohlraum der Zunge herausziehbar sind.

Die Kernteile sind dabei massiv, vorzugsweise aus Gips, ausgebildet.

Ebenso erscheint es nach der Erfindung möglich, die Kernteile bei gemeinsamer Außenkontur mit einem durch Federwirkung überbrückten Spiel gegeneinander auszubilden, so daß zum Ziehen der Kernteile eine Relativbewegung der Kernteile zueinander möglich ist.

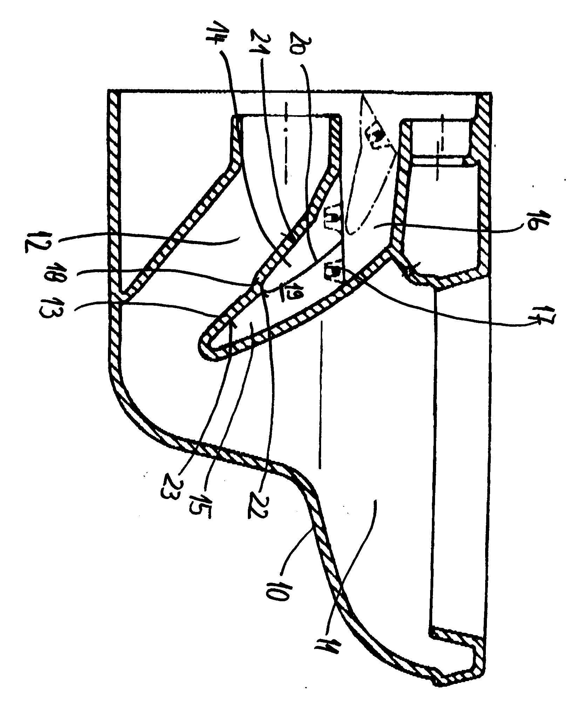

In der Zeichnung ist ein Ausführungsbeispiel der Erfindung wiedergegeben, welches nachstehend beschrieben ist. Die einzige Figur zeigt einen Schnitt durch ein im Hohlguß gefertigtes Tiefspülklosett mit belassenen Kernteilen in dem Zungenhohlraum, wobei das Herausziehen des ersten, kleineren Kernteils strichpunktiert angedeutet ist.

Zwischen dem Becken 11 und dem Spülkanal 12 eines durch Scherbenbildung hergestellten Tiefspülklosetts 10 ist eine Geruchsverschlußzunge 13 angeordnet, die nach Durchführung des erfindungsgemäßen Verfahrens doppelwandig ausgebildet ist. Diese Ausführung ist hervorgerufen durch den Einsatz von zwei Kernteilen unterschiedlicher Größe, von denen ein kleineres Kernteil mit 14 und ein größeres Kernteil mit 15 bezeichnet sind. Die Kernteile 14,15 sind bei der Vorbereitung der Gießform jedes für sich mit einem die aus der Figur ersichtliche Öffnung 16 ausbildenden Rückwandkern verbindbar, der in dem aus der Zeichnung ersichtlichen Verfahrensabschnitt bereits vollständig gezogen ist.

Wie sich aus der Zeichnung ergibt, erlaubt die freie Öffnung 16 den Zugriff auf die nach Entfernung des Rückwandkerns in der Zunge 13 noch befindlichen Kernteile 14,15, wobei in der Zeichnung strichpunktiert das Herausziehen des kleineren Kernteils 14 bereits angedeutet ist. Die beiden Kernteile 14,15 bilden dabei gemeinsam die Kontur einer Geruchsverschlußzunge 13 aus, wobei die Kernteile 14,15 jedes für sich über eine Gestaltung 17 lösbar an dem nicht dargestellten, die Öffnung 16 ausfüllenden Rückwandkern befestigbar sind.

Damit der zuerst zu entfernende kleinere Kernteil 14 aus dem durch den Scherben 18 gebildeten inneren Zungenhohlraum 19 herausziehbar ist, weist der kleinere Kernteil 14 eine hier nur etwa halb so große Länge auf wie dies bei dem größeren Kernteil 15 der Fall ist, so daß der größere Kernteil 15 mit seinem vorderen Bereich die Zungenkontur vollständig ausbildet. Die dem Kernteil 15 zugewandte Fläche 20 des Kernteiles 14 und damit die Verbindungsfläche zwischen den beiden Kernteilen 14,15 hat einen in Auszugsrichtung bogenförmig gekrümmten Verlauf, der gemeinsam mit der kleineren Länge des Kernteils 14 die Überwindung des zwischen der Zunge und der durch den Rückwandkern gebildeten Öffnung 16 gegebenen Winkels durch Herausdrehen des kleineren Kernteils 14 erleichtert.

Der Erleichterung des Herausdrehens dient auch der aus der Zeichnung ersichtliche flachere Winkel, den die äußere Fläche 21 des kleineren Kernteils 14 mit der Öffnung 16 bildet, wobei unter Ausbildung einer Stufe 22 der größere Kernteil 15 mit seinem vorderen Ende (Fläche 23) einen steileren Verlauf der Zungenwandung bestimmt.

## Patentansprüche

1. Gießform zur Herstellung eines Tiefspülklosetts (10) mit einer von der von der Rückwand zum Becken (11) einspringenden Öffnung (16) ausgehenden Geruchsverschlußzunge (13) im Hohlguß mit einem Rückwandkern zur Ausbildung der Öffnung (16) und mit wenigstens einem Formteil zur Ausbildung der Geruchsvershlußzunge (13), dadurch gekennzeichnet, daß das Formteil aus mindestens zwei zusammengesetzt die Kontur der Geruchsverschlußzunge (13) ausbildenden Kernteilen (14, 15) unterschiedlicher Abmessung besteht, die in einander angepaßter Formgebung derart ausgebildet sind, daß ein erstes kleineres Kernteil (14) aus dem durch Scherbenbildung gebildeten Zungenhohlraum (19) bei dem dort noch verbleibenden zweiten größeren Kernteil (15) herausziehbar ist, wobei die Berührungsfläche (20) zwischen den Kernteilen (14, 15) eine in Richtung des Ausziehweges für die Kernteile (14, 15) verlaufende Krümmung aufweist.

2. Gießform nach Anspruch 1, dadurch gekennzeichnet, daß die Länge des zuerst auszuziehenden kleineren Kernteils (14) kürzer bemessen ist als die Länge des zweiten größeren Kernteils (15).

3. Gießform nach Anspruch 2, dadurch gekennzeichnet, daß die Länge des kleineren Kernteils (14) zwischen einem Drittel und zwei Dritteln der Länge des größeren Kernteils (15) beträgt.

4. Gießform nach einem der Ansprüche 1 bis 3, dadurch gekennzeichnet, daß die zum Rückwandkern gerichtete, aus den Flächen (21,23) der zusammengesetzten Kernteile (14,15) gebildete Fläche der Zunge (13) unter einem flacheren Winkel zum Rückwandkern verläuft als die gegenüberliegende dem Becken (11) zugewandte Zungenfläche.

5. Gießform nach einem der Ansprüche 1 bis 4, dadurch gekennzeichnet, daß die zum Rückwandkern gerichtete Fläche (21) des zuerst auszuziehenden kleineren Kernteils (14) mit diesem einen flacheren Winkel als die die Fortsetzung des ersten Kernteils (14) bildende, zum Rückwandkern gerichtete Fläche (23) des zweiten größeren Kernteils bildet.

6. Gießform nach einem der Ansprüche 1 bis 5, dadurch gekennzeichnet, daß drei ineinander einsetzbare und eine gemeinsame Kontur ausbildende Kernteile vorgesehen sind.

7. Gießform nach einem der Ansprüche 1 bis 6, dadurch gekennzeichnet, daß die Kernteile (14,15) massiv, vorzugsweise aus Gips, ausgebildet sind.

8. Gießform nach Anspruch 1, dadurch gekennzeichnet, daß die Kernteile (14,15) bei gemeinsamer Außenkontur ein durch Federwirkung überbrückbares Spiel gegeneinander aufweisen.

## Claims

1. Casting mould for producing a flush-down type water closet (10) having a stench trap tongue (13) which issues from the opening (16), re-entering from the rear wall to the bowl (11), in the hollow casting with a rear wall core for the formation of the opening (16) and with at least one mould portion for the formation of the stench trap tongue (13), characterised in that the mould portion consists of at least two core portions (14, 15) of differing dimension which when put together form the contour of the stench trap tongue (13) and which are formed so as to be shaped to match each other in such a way that a first smaller core portion (14) can be drawn out of the tongue hollow space (19) formed by formation of the Ceramic body with the second larger core portion (15) still remaining there, in which the contact surface (20) between the core portions (14, 15) has a curvature which extends in the direction of the drawing-out path for the core portions (14, 15).

2. Casting mould according to claim 1, characterised in that the length of the smaller core portion (14) to be drawn out first is dimensioned so as to be shorter than the length of the second larger core portion (15).

3. Casting mould according to claim 2, characterised in that the length of the smaller core portion (14) amounts to between one third and two thirds of the length of the larger core portion (15).

4. Casting mould according to one of the claims 1 to 3, characterised in that the surface of the tongue (13), which is directed towards the rear wall core and which is formed from the surfaces (21, 23) of the core portions (14, 15) put together, extends at a lower angle relative to the rear wall core than the opposing tongue surface facing the bowl (11).

5. Casting mould according to one of the claims 1 to 4, characterised in that the surface (21), directed towards the rear wall core, of the smaller core portion (14) to be drawn out first forms with the latter a lower angle than the surface (23) of the second larger core portion, which surface (23) constitutes the continuation of the first core portion (14) and is directed towards the rear wall core.

6. Casting mould according to one of the claims 1 to 5, characterised in that three core portions are provided, which portions can be inserted into each other and form a common contour.

7. Casting mould according to one of the claims 1 to 6, characterised in that the core portions (14, 15) are formed so as to be solid, preferably of gypsum.

8. Casting mould according to claim 1, characterised in that the core portions (14, 15) with common outer contour have a clearance in respect of each other which can be bridged by spring action.

## Revendications

1. Moule de coulée pour la fabrication d'une cuvette de WC à action siphonique (10), avec une languette d'obturation (13) contre les odeurs, partant d'une ouverture (16), formée à partir de la paroi arrière de la cuvette (11) et réalisée en moulage creux, avec un noyau de paroi arrière destiné à configurer l'ouverture (16) et avec au moins une pièce profilée, destinée à configurer la languette d'obturation contre les odeurs (13), caractérisé en ce que la pièce profilée est composée d'au moins deux parties de noyau (14, 15) de dimensions différentes, constituant, une fois assemblées, le contour de la languette d'obturation contre les odeurs (13) et dont la configuration, mutuellement adaptée, est réalisée de telle façon qu'une première plus petite partie de noyau (14), est susceptible d'être extraite de l'espace creux de languette (19), formé par de la pâte, alors que la deuxième partie de noyau (15) plus grande reste encore en place, la surface de contact (20) entre les parties de noyau (14, 15) présentant une courbure évoluant en direction du chemin d'extraction des parties de noyau (14, 15).

2. Moule de coulée selon la revendication 1, caractérisé en ce que la longueur de la plus petite partie de noyau (14) devant être extraite en premier est inférieure à la longueur de la deuxième plus grande partie de noyau (15).

3. Moule de coulée selon la revendication 2, caractérisé en ce que la longueur de la plus petite partie de noyau (14) est comprise entre un tiers et deux tiers de la longueur de la plus grande partie de noyau (15).

4. Moule de coulée selon l'une des revendications 1 à 3, caractérisé en ce que la surface de languette (13), orientée vers le noyau de paroi arrière et formée à partir des surfaces (21, 23) des parties de noyau (14, 15) assemblées, s'étend suivant un angle plus plat, par rapport au noyau de paroi arrière, que la surface de languette opposée tournée vers la cuvette (11).

5. Moule de coulée selon l'une des revendications 1 à 4, caractérisé en ce que la surface (21), orientée vers le noyau de paroi arrière, de la petite paroi de noyau (14) à extraire en premier, forme avec celle-ci un angle plus plat que la surface (23), délimitant le prolongement de la première partie de noyau (14) et orientée vers le noyau de paroi arrière, de la deuxième partie de noyau plus grande.

6. Moule de coulée selon l'une des revendications 1 à 5, caractérisé en ce que sont prévues trois parties de noyau, insérables les unes dans les autres et formant un contour commun.

7. Moule de coulée selon l'une des revendications 1 à 6, caractérisé en ce que les parties de noyau (14, 15) sont de réalisation massive, de préférence en gypse.

8. Moule de coulée selon la revendication 1, caractérisé en ce que les parties de noyau (14, 15) présentent, les unes par rapport aux autres, un jeu pouvant être compensé par effet élastique, tout en ayant un contour extérieur commun.
